# EUROPEAN PATENT SPECIFICATION

(11) **EP 2 409 659 B1**
(45) Date of publication and mention of the grant of the patent: **02.12.2015**
(21) Application number: 11170958.0
(22) Date of filing: 22.06.2011
(51) Int. Cl.: A61B 18/00, A61B 18/14, A61B 18/04

(54) **Improved electrosurgical electrodes and materials**
Verbesserte elektrochirurgische Elektroden und Materialien
Électrodes électrochirurgicales améliorées et matériaux

(30) Priority: 23.06.2010 US 821898
(43) Date of publication of application: 25.01.2012
(73) Proprietor: Covidien LP, Mansfield, MA 02048 (US)
(72) Inventor: Keppel, David S., Longmont, CO, 80501 (US)
(74) Representative: Soames, Candida Jane

(56) References cited:
- EP-A1- 2 153 789
- WO-A1-02/30308
- WO-A1-2006/084316

## Description

### BACKGROUND

### 1. Technical Field

The present disclosure relates generally to electrosurgical systems suitable for cutting and/or coagulating tissue. More particularly, the present disclosure is directed to an electrode having a low work function suitable for use in a monopolar electrosurgical procedure.

### 2. Background of the Related Art

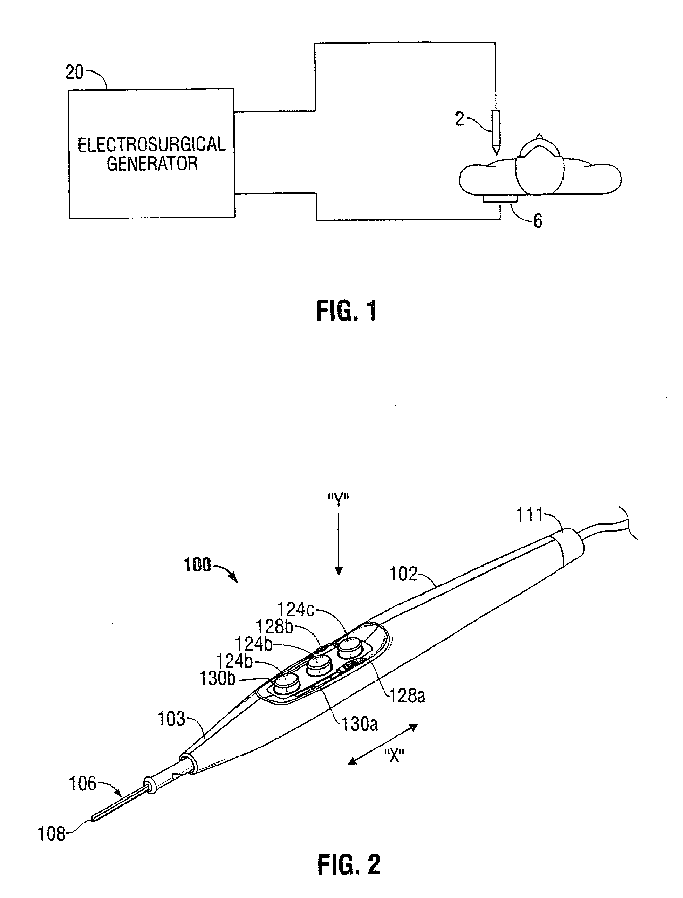

Energy-based tissue treatment is well known in the art. Various types of energy (e.g., electrical, ultrasonic, microwave, cryogenic, thermal, laser, etc.) are applied to tissue to achieve a desired result. Electrosurgery involves application of high radio frequency electrical current to a surgical site to cut, ablate, coagulate or seal tissue. In monopolar electrosurgery, as shown in Fig. 1, a source or active electrode 2 delivers radio frequency energy from the electrosurgical generator 20 to the tissue and a return electrode 6 carries the current back to the generator. In monopolar electrosurgery, the source electrode is typically part of the surgical instrument held by the surgeon and applied to the tissue to be treated. A patient return electrode is placed remotely from the active electrode to carry the current back to the generator.

Electrosurgical instruments have become widely used by surgeons in recent years. By and large, most electrosurgical instruments are hand-held instruments, e.g., an electrosurgical pencil, which transfer radio-frequency (RF) electrical or electrosurgical energy to a tissue site. As used herein the term "electrosurgical pencil" is intended to include instruments which have a handpiece that is attached to an active electrode and which is used to cauterize, coagulate and/or cut tissue. Typically, the electrosurgical pencil may be operated by a handswitch or a footswitch. The active electrode is an electrically conducting element that is usually elongated and may be in the form of a thin flat blade with a pointed or rounded distal end. Alternatively, the active electrode may include an elongated narrow cylindrical needle that is solid or hollow with a flat, rounded, pointed or slanted distal end. Typically electrodes of this sort are known in the art as "blade", "loop" or "snare", "needle" or "ball" electrodes.

As mentioned above, the handpiece of the electrosurgical pencil is connected to a suitable electrosurgical energy source (i.e., generator) which produces the radio-frequency electrical energy necessary for the operation of the electrosurgical pencil. In general, when an operation is performed on a patient with an electrosurgical pencil, electrical energy from the electrosurgical generator is conducted through the active electrode to the tissue at the site of the operation and then through the patient to a return electrode. The return electrode is typically placed at a convenient place on the patient's body and is attached to the generator by a conductive material.

A key to electrosurgery, for example, both cutting and coagulating, is forming discharge plasma at the active electrode. In order to form discharge plasma, there must be sufficient voltage. There are two different types of discharge: glow discharge and thermal arc. In order to transition from glow to thermal arc discharge, there must be sufficient free electrons to sustain the arc. These electrons come from one of the electrodes (either tissue or active electrode) by the mechanism of thermionic emission or field emission. Tissue is not a good source of electrons; hence the bulk of electrons for arcing come from the active electrode. The ability of an electrode to provide electrons is key to creating an arc and sustaining the arc. Several different mechanisms come into play when determining a materials ability to source electrons which include, Richardsons Law, Sommerfeld Formula, the Schottky Effect and Field Emission (Fowler Nordheim). Such mechanisms indicate that lowering the work function of a material or increasing the field strength will increase the amount of electrons available. "Work function" is the ability of a material to provide electrons and is measured as the energy level required for removing an electron from a solid to a point immediately outside the solid surface. Conventional electrodes use material that has a high work function such as pure tungsten that is approximately 4.5 electron volts (eV). Using a material with a high work function in medical application is typically not desirable because a high work function requires a higher voltage to form a plasma discharge.

### SUMMARY

The invention is set forth in the appended claims.

In an embodiment of the present invention, an electrosurgical instrument is provided having a hand-held applicator having proximal and distal ends and a gas delivery member adapted to deliver pressurized ionizable gas to the proximity of a tungsten alloy electrode located adjacent the distal end of the applicator. The tungsten alloy electrode is configured to connect to a source of electrosurgical energy. The electrosurgical instrument may also include an actuator assembly configured to selectively receive a source of pressurized ionizable gas therein and having at least one controller that controls the delivery of the gas from the supply of pressurized inert gas to the hand-held applicator and controls the delivery of electrosurgical energy to the hand-held appl icator;

The tungsten alloy electrode may include an oxide having at least lanthanum, cerium and/or zirconium. The tungsten alloy electrode may also have a work function less than 4.5 electron volts (eV).

### BRIEF DESCRIPTION OF THE DRAWINGS

The above and other aspects, features, and advantages of the present disclosure will become more apparent in light of the following detailed description when taken in conjunction with the accompanying drawings in which:
Fig. 1 is a schematic diagram of an electrosurgical system;
Fig. 2 is a perspective view of an electrosurgical pencil according to an embodiment of the present disclosure;
Figs. 3A-3E are side views of end effectors adapted for use with the electrosurgical pencil of Fig. 2 in accordance with embodiments of the present disclosure;
Fig. 4 is a schematic, side view of a gas-enhanced electrosurgical instrument according to an embodiment of the present disclosure;
Figs. 5A-5D are side views of needles adapted for use with the gas enhanced electrosurgical instrument of Fig. 4 according to embodiments of the present disclosure; and
Fig. 6 is a schematic energy diagram according to an embodiment of the present disclosure.

### DETAILED DESCRIPTION

Particular embodiments of the present disclosure are described hereinbelow with reference to the accompanying drawings; however, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

Like reference numerals may refer to similar or identical elements throughout the description of the figures. As shown in the drawings and described throughout the following description, as is traditional when referring to relative positioning on a surgical instrument, the term "proximal" refers to the end of the apparatus which is closer to the user and the term "distal" refers to the end of the apparatus which is further away from the user.

Electromagnetic energy is generally classified by increasing energy or decreasing wavelength into radio waves, microwaves, infrared, visible light, ultraviolet, X-rays and gamma-rays. As used herein, the term "microwave" generally refers to electromagnetic waves in the frequency range of 300 megahertz (MHz) (3 x 10⁸ cycles/second) to 300 gigahertz (GHz) (3 x 10¹¹ cycles/second). As used herein, the term "RF" generally refers to electromagnetic waves having a lower frequency than microwaves.

Turning to Fig. 2, an electrosurgical pencil constructed in accordance with an embodiment of the present disclosure is shown generally as 100. It should be understood, that electrosurgical pencil 100 is merely illustrative of a type of apparatus that can be used in accordance with embodiments described herein. Any electrosurgical pencil may be used in accordance with embodiments described herein.

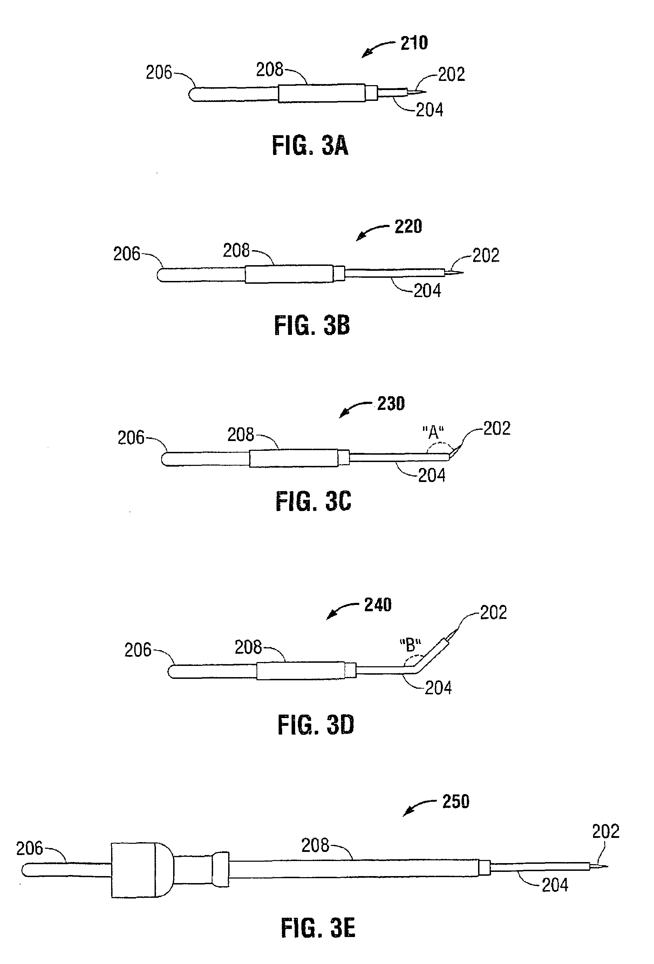

Electrosurgical pencil 100 includes an elongated housing 102 configured and adapted to support a blade receptacle 104 at a distal end 103 thereof which, in turn, receives a replaceable electrocautery end effector 106 that will be described hereinbelow with regard to Figs. 3A-3E. A distal end portion 108 of end effector 106 extends distally from receptacle 104 while a proximal end portion of blade 106 is retained within distal end 103 of housing 102.

Electrosurgical pencil 100 includes at least one activation switch, preferably three activation switches 124a-124c, each of which are supported on an outer surface 107 of housing 102. Each activation switch 124a-124c is operatively connected to a respective switch 126a-126c which, in turn, controls the transmission of RF electrical energy supplied from a generator to end effector 106. Activation switches 124a-124c are configured and adapted to control the mode and/or "waveform duty cycle" to achieve a desired surgical intent. Electrosurgical pencil 100 further includes at least one intensity controller 128a and/or 128b, each of which are slidingly supported in guide channels 130a, 130b, respectively, which are formed in outer surface 107 of housing 102. Each intensity controller 128a and 128b is a slide-like potentiometer. Intensity controllers 128a and 128b are configured and adapted to adjust one of the power parameters (e.g., voltage, power and/or current intensity) and/or the power verses impedance curve shape to affect the perceived output intensity. Activation switches 124a-124c and intensity controllers 128a and 128b control the output of RF energy from end effector 106.

A proximal portion 111 of housing 102 has a transmission line or cable 112 attached thereto. Cable 122 may be connected to an electrosurgical generator (not shown) or a return pad (not shown). Alternatively, an electrosurgical generator may be included in housing 102.

Figs. 3A-3E depict various end effectors 210, 220, 230, 240 and 250 suitable for use with an electrosurgical pencil as described above. Each of the end effectors includes a proximal end 206 that is retained within a distal end of an electrosurgical pencil. High temperature insulation 204 is provided to guard against accidental tissue contact and/or burns. Insulation 204 may be made from high temperature thermoplastics such as PEEK or from thermosets such as PTFE. At the distal end of the end effectors is a tip 202 formed from a tungsten alloy that will be described hereinbelow. The end effector may also have a safety sleeve 208, made for example of polyolefin shrink tubing, that reduces the risk of alternate site burns. As can be seen in Figs. 3A-3E, the end effectors 210, 220, 230, 240 and 250 may come in various lengths and angles. For instance, the tip 202 may be bent at an angle "A" between 0° and 180° as shown in Fig. 3C or a portion along the insulation 204 may be bent at an angle "B" between 0° and 180°. A user can select the appropriate length and angle based on the user's particular needs at any given moment.

The small diameter of the tip 202 restricts the tips ability to cool itself thereby lowering he work function of the tip 202. Further, the sharp point of the needle lowers the work function due to the Schottky or field emission effects. By using a tungsten alloy instead of a pure tungsten, the work function of the tip 202 is lowered even further.

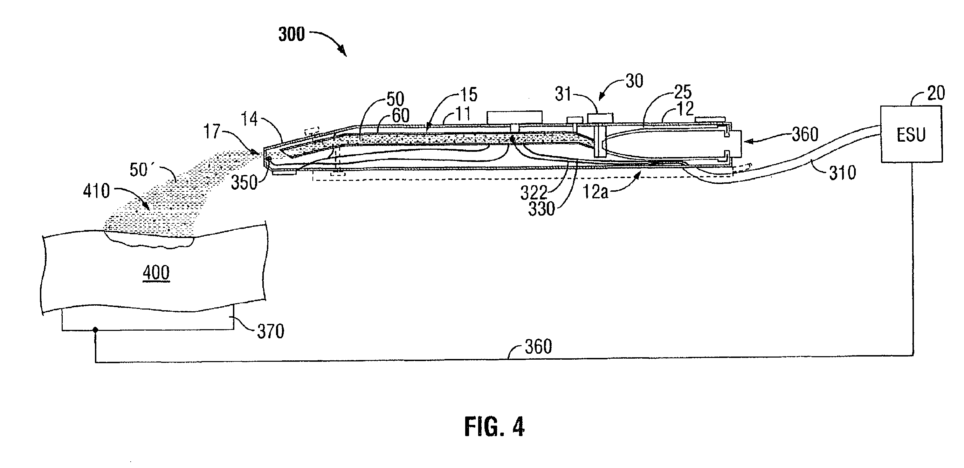

Fig. 4 depicts a schematic, side view of a gas-enhanced electrosurgical instrument 300 according to an embodiment of the present disclosure. While the following description will be directed towards electrosurgical instruments, the features and concepts (or portions thereof) of the present disclosure can be applied to any type of electrosurgical instrument, e.g., forceps, suction coagulators, vessel sealers, etc. The electrosurgical type instrument may be monopolar or bipolar as appropriate.

Referring to Fig. 4, electrosurgical instrument or coagulator 300 is dimensioned to be pencil-like or hand-held and is configured for use during open surgical procedures, A similar instrument or coagulator may be configured, for example, with a pistol grip or handle dimensioned for laparoscopic or endoscopic surgical procedures. Further, although the basic operating features of an open electrosurgical coagulator 300 are described herein, the same or similar operating features may be employed on or used in connection with a laparoscopic or endoscopic electrosurgical coagulator or instrument, manually or robotically operated, without departing from the scope of the present disclosure.

As shown in Fig. 4, coagulator 300 includes a frame, shown as an elongated housing 11, having a proximal end 12, a distal end 14 and an elongated cavity 15 extending therethrough, for supporting and/or housing a plurality of internal and/or external mechanical and electromechanical components thereon and therein.

Distal end 14 of housing 11 includes a distal port 17 which is designed to emit, expel or disperse gas emanating from an elongated gas supply channel or tube 60 that runs generally longitudinally through the frame or housing 11 of coagulator 300. Tube 60 is for supplying pressurized gas 50 to the proximity of an active electrode 350 located adjacent distal end 14 of housing 11. Electrode 350 is proximal of port 17 such that the gas that is emitted from port 17 is ionized. Elongated housing 11 includes a receptacle 25, typically positioned adjacent the proximal end 12 that may be part of a unitary or integral handle portion 12a of housing 11. Receptacle 25 is dimensioned to securely engage and receive or seat a gas pressurized container, canister, cartridge or cylinder 360 therein. Cylinder 360 contains a surgical gas, e.g., a noble or inert gas, or mixture of noble or inert gases. References herein to inert gas or gases are understood to include noble gas or gases. The preferred inert gas is argon. Cylinder 360 is relatively small, single use and disposable. The cylinder is of standardized design and certified for transportation requirements. Alternatively, a gas supply line may be utilized from a remote gas source located outside the operating room.

Elongated gas supply tube 60 is adapted and dimensioned to channel or carry pressurized gas 50 from cylinder 360 through a regulator or valve 30 to or through distal end 14 of coagulator 300 for ionization, typically prior to the gas emitting and dispersing from distal port 17. Regulator or valve 30 can be part of or attached to cylinder 360, housing 11, or actuator assembly 31. It is envisioned that distal port 17 or distal end 14 may be configured to facilitate or promote the dispersion of the ionized gas plasma 50' from distal port 17 in a uniform and consistent manner. For example, distal end 14 may be tapered on one, both or all sides thereof to direct the ionized plasma 50' toward a surgical or operative site 410. Alternatively, distal port 17 may be configured to disrupt or aggravate the dispersion or flow of gas plasma 50' exiting distal port 17 to enhance coagulation by creating a more turbulent gas flow. Many suitable devices, e.g., screws, fans, blades, helical patterns, etc., may be employed to cause gas plasma 50' to flow more or less turbulently or with other predetermined flow characteristics through tube 60 and/or out of distal port 17.

Elongated housing 11 is connected, for example, by an electrical cable 310, to a source of electromagnetic energy generally designated ESU, e.g., an electrosurgical generator 20. As mentioned above, proximal end 12 includes a receptacle 25 which receives, securely engages and seats cylinder 360 therein. Receptacle 25 and/or cylinder 360 need not be, as in the case of a single use disposable instrument, but may be configured to allow cylinder 360 to be selectively removable and replaceable within receptacle 25. For example, proximal end 12 of elongated housing 11, or receptacle 25 may include a locking mechanism 40 which upon insertion of a cylinder 360 into receptacle 25 automatically (or manually) releasably locks the cylinder 360 securely within receptacle 25. By unlocking locking mechanism 40, cylinder 360 may be removed and replaced with another cylinder 360.

Electrosurgical instrument 300 includes at least one actuator assembly, e.g., a dial or button, generally designated 31, for actuating and selectively adjusting the flow of pressurized inert gas 50 from cylinder 360 to the proximity of active electrode 350, and for actuating and selectively adjusting the delivery of electrosurgical energy from the source, i.e., from generator 20, through cable 310 and leads 322, 330, to the active electrode 350 for ionizing the inert gas for use at the surgical site 410. Actuator assembly 31 can also operate as the actuator for actuating delivery of electrosurgical energy from the source.

A return electrode or pad 370 is typically positioned under the patient and connected to a different electrical potential on electrosurgical generator 300 via cable 360. During activation, return pad 370 acts as an electrical return for the electrosurgical energy emanating from electrosurgical coagulator 300.

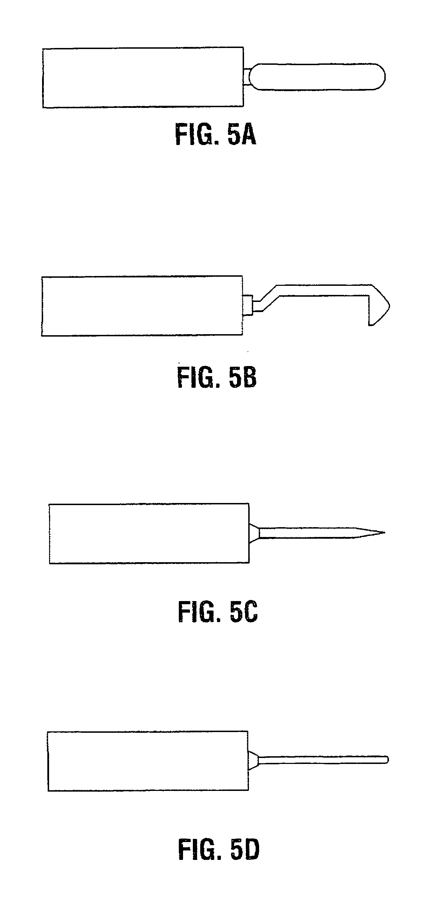

Active electrode 350 can be attached to or mechanically engaged with the distal end of the housing and positioned adjacent to or at an operating site 410. Active electrode 350 is positioned adjacent the distal end of frame or housing 11 between the distal end of tube 60 and distal port 17, although the active electrode can be located just to the exterior of port 17. For example, active electrode 350 can be mounted to an elongated member that is supported within housing 11 and that extends outside of the housing, such that the electrode is positioned just outside of the port. Figs. 5A-5D depict examples of active electrode 350 that may be used with electrosurgical instrument 300. As shown in Figs. 5A-5D, active electrode may be a blade (Fig. 5A), L-shaped (Fig. 5B), a sharp needle (Fig. 5C) or a blunt needle (Fig. 5D). The active electrode 350 is made from a tungsten alloy that will be described hereinbelow.

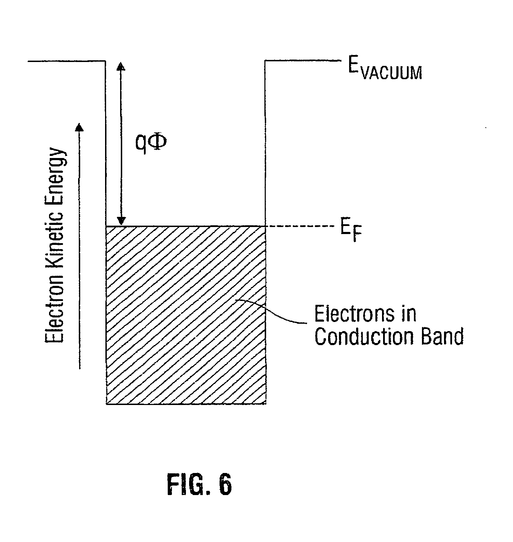

As discussed above, tips 202 (as shown in Figs. 3A-3E) and active electrodes 350 (as shown in Figs. 5A-5D) are made from a tungsten alloy. In order to understand and appreciate the possibilities of using a tungsten alloy work function needs to be defined. The work function is generally defined as the minimum amount of energy needed to remove an electron from the metal and is measured in electron volts (eV). The work function is a material constant for certain metals. For instance, the work function for pure tungsten is approximately 4.5 eV. As shown in the free electron model of Fig. 6, at a lower electron kinetic energy, electrons exist in a conduction band. Energy possessed by electrons in the highest occupied level is known as the Fermi energy (E_{F}). In order for electrons to be free from the metal, the electrons have to acquire work function energy (qΦ) to reach the vacuum level (Eᵥ). To this end, the lower the work function of an electrode, the lower the voltage necessary to strike an arc, hence the easier the arc initiates. Conversely, the higher the work function requires a higher voltage necessary to strike an arc.

In order to lower the work function of the tips 202 or electrodes 350, tungsten is alloyed with another material such as oxides of Cerium, Lanthanum and/or Zirconium. Alloying the tungsten with such oxides lowers the work function to below 4.5 eV. In operation, when the surgeon activates the electrosurgical pencil 100 or electrosurgical coagulator 300, the oxides in the tungsten alloy migrate from inside the tungsten to the point of the tip 202 or electrode 350 which gives off the oxide element in the air and leaves a film of the metal alloy on the tip. This causes the tip 202 or electrode 350 to have a different temperature at the tip based on the work function of that element. The oxides that are emitted at the tip serve to improve arc initiation and stability. The oxides also cause the tip 202 or electrode 350 to provide the same level of emission as a pure tungsten but at much lower temperatures. The lower temperatures improve the longevity of the tip 202 or electrode 350.

Using a material with a low work function is desirable in medical applications because a low work function requires a lower voltage to form a plasma discharge or to strike an arc. Therefore, by lowering the work function of the tip 202 or electrode 350, the voltage required to initiate an arc is lowered. A lower voltage setting is especially advantageous in argon enhance electrosurgery, which requires leads to start from a greater distance to tissue. When used with micro-needles, the needle does not need to be as sharp to take advantage of the Schottky effect that lowers work function by concentrating the electric field. Further, since the lowered work function materials remain cooler than higher work function materials, a longer electrode life can be anticipated. Additionally, arc stability is improved using a lower work function material because a surgeon has more control over the surgical effect.

The tungsten alloy tip 202 or tungsten alloy electrode 350 may be alloyed with other elements such as lanthanum, cerium, zirconium or the like, in order to improve the flexibility and/or lower the cost of the tip 202 or electrode 350.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

## Claims

1. An electrosurgical instrument, comprising:
a hand-held applicator having proximal and distal ends;
an electrode located adjacent the distal end of the applicator and adapted to connect to a source of electrosurgical energy;
the hand-held applicator having a gas delivery member adapted to deliver pressurized ionizable gas to the proximity of the electrode; and
an actuator assembly configured to selectively receive a source of pressurized ionizable gas therein and having at least one controller that controls the delivery of the gas from the supply of pressurized inert gas to the hand-held applicator and controls the delivery of electrosurgical energy to the hand-held applicator; **characterised in that**:
the electrode is a tungsten alloy electrode.

2. The electrosurgical instrument of claim 1, wherein the tungsten alloy electrode includes an oxide having at least lanthanum.

3. The electrosurgical instrument according to claim 1, wherein the tungsten alloy electrode includes an oxide having at least cerium.

4. The electrosurgical instrument according to claim, wherein the tungsten alloy electrode includes an oxide having at least zirconium.

5. The electrosurgical instrument of any one of claims 1 to 4, wherein the tungsten alloy electrode has a work function less than about 4.5 electron volts (eV).

## Patentansprüche

1. Ein elektrochirurgisches Instrument, aufweisend:
einen in der Hand gehaltenen Applikator mit proximalen und distalen Enden;
eine Elektrode, die sich benachbart zu dem distalen Ende von dem Applikator befindet und die angepasst ist zum Verbinden mit einer Quelle von elektrochirurgischer Energie;
wobei der in der Hand gehaltene Applikator ein Gaslieferungselement hat, das angepasst ist zum Liefern von unter Druck stehendem ionisierbaren Gas in die Nähe der Elektrode; und
eine Aktuatoranordnung, die konfiguriert ist zum selektiven darin Empfangen einer Quelle von unter Druck stehendem ionisierbaren Gas und mit wenigstens einem Steuerer, welcher die Lieferung von dem Gas von der Einspeisung von unter Druck stehendem Inertgas zu dem in der Hand gehaltenen Applikator steuert und die Lieferung von elektrochirurgischer Energie zu dem in der Hand gehaltenen Applikator steuert, **dadurch gekennzeichnet, dass**:
die Elektrode eine Wolfram-Legierung-Elektrode ist.

2. Das elektrochirurgisches Instrument von Anspruch 1, wobei die Wolfram-Legierung-Elektrode ein Oxid mit wenigstens Lanthan umfasst.

3. Das elektrochirurgisches Instrument gemäß Anspruch 1, wobei die Wolfram-Legierung-Elektrode ein Oxid mit wenigstens Cerium umfasst.

4. Das elektrochirurgisches Instrument gemäß Anspruch 1, wobei die Wolfram-Legierung-Elektrode ein Oxid mit wenigstens Zirconium umfasst.

5. Das elektrochirurgisches Instrument gemäß einem der Ansprüche 1 bis 4, wobei die Wolfram-Legierung-Elektrode eine Austrittsarbeit von weniger als etwa 4,5 Elektronenvolt (eV) hat.

## Revendications

1. Instrument électrochirurgical, comprenant :
un applicateur portatif possédant des extrémités proximale et distale ;
une électrode placée de façon adjacente à l'extrémité distale de l'applicateur et conçue pour être connectée à une source d'énergie électrochirurgicale ;
l'applicateur portatif possédant un élément de distribution de gaz conçu pour distribuer du gaz ionisable sous pression à proximité de l'électrode ; et
un ensemble actionneur configuré pour recevoir sélectivement une source de gaz ionisable sous pression à l'intérieur et possédant au moins un régulateur qui régule la distribution du gaz de la réserve de gaz inerte sous pression à l'applicateur portatif et régule la distribution d'énergie électrochirurgicale à l'applicateur portatif ; **caractérisé en ce que** :
l'électrode est une électrode en alliage de tungstène.

2. Instrument électrochirurgical selon la revendication 1, dans lequel l'électrode en alliage de tungstène inclut un oxyde comportant au moins du lanthane.

3. Instrument électrochirurgical selon la revendication 1, dans lequel l'électrode en alliage de tungstène inclut un oxyde comportant au moins du cérium.

4. Instrument électrochirurgical selon la revendication 1, dans lequel l'électrode en alliage de tungstène inclut un oxyde comportant au moins du zirconium.

5. Instrument chirurgical selon l'une quelconque des revendications 1 à 4, dans lequel l'électrode en alliage de tungstène a un potentiel d'extraction inférieur à environ 4,5 électron-volts (eV).
